# EUROPEAN PATENT APPLICATION

(11) **EP 4 389 278 A1**
(43) Date of publication of application: **26.06.2024**
(21) Application number: 22215534.3
(22) Date of filing: 21.12.2022
(51) Int. Cl.: B01J 23/755, B01J 38/12, B01J 38/48, C07C 2/10

(54) **REGENERATION OF A NICKEL CONTAINING OLEFIN OLIGOMERIZATION CATALYST**

(71) Applicant: BASF SE, 67056 Ludwigshafen am Rhein (DE)
(72) Inventor: WLOKA, Veronika, 67056 Ludwigshafen am Rhein (DE); HEIDEMANS, Thomas, 67056 Ludwigshafen am Rhein (DE); LOTTENBURGER, Ines, 67056 Ludwigshafen am Rhein (DE); STOECKLE, Stefan, 67056 Ludwigshafen am Rhein (DE)
(74) Representative: BASF IP Association

(57) **Abstract**

The invention concerns a process for regenerating a used catalyst which has been used for the oligomerization of C3 to C6 olefins, the catalyst in the fresh state having had an active material composition of 10 to 70 % by weight of NiO, 5 to 30 % by weight TiO₂ and/or ZrO₂, 0 to 20 % by weight Al₂O₃ and the balance to 100 % by weight SiO₂, comprising the steps of a) calcining the used catalyst at a temperature of 300 to 600°C in the presence of an oxygen containing gas, b) applying nickel in an amount corresponding to from 1 to 5 % by weight of the used catalyst mass to the catalyst obtained in step a), and c) drying and calcining the catalyst obtained in step b).

## Description

### TECHNICAL FIELD

The invention concerns a process for regenerating a used catalyst which has been used for the oligomerization of C3 to C6 olefins, the catalyst in the fresh state having had an active material composition of 10 to 70 % by weight of NiO, 5 to 30 % by weight TiO₂ and/or ZrO₂, 0 to 20 % by weight Al₂O₃ and the balance to 100 % by weight SiO₂.

### BACKGROUND ART

Olefins having from 3 to 6 carbon atoms or mixtures thereof are available in large quantities both from FCC (Fluidized Catalyst Cracking) plants and from steam crackers. They are used in various applications in industrial scale, for example for the oligomerization or co-oligomerization of the olefins to higher olefins. Among the C3 to C6 olefins, in particular the C4 fraction, i.e. a mixture comprising essentially butenes and butanes, if appropriate after removal of isobutene, is known to be suitable for the preparation of oligomers, in particular octenes and dodecenes. Both the octenes and the dodecenes can, after hydroformylation and subsequent hydrogenation to the corresponding alcohols, be used, for example, for preparing plasticizers or surfactants.

The degree of branching of the olefins plays a critical role for some applications, for example for the use of olefins as plasticizers or surfactant alcohols. The degree of branching is described, for example, by the ISO index which indicates the mean number of alkyl branches of the respective olefin fraction. As an example, in a C8 fraction, n-octenes contribute 0, methylheptenes contribute 1, dimethylhexenes contribute 2 and trimethyl-pentenes contribute 3 to the ISO index of the fraction. As a further example, in a C12 fraction, n-dodecenes contribute 0, methylundecenes contribute 1 and dimethyldecenes contribute 2 to the ISO index of the fraction. The lower the ISO index, the higher the linearity of the molecules in the respective fraction, and with that the higher the yields in the hydroformylation and the better the properties of the plasticizer or surfactants prepared therewith.

The oligomerization is carried out industrially under either homogeneous or heterogeneous catalysis. The homogeneously catalyzed process has the disadvantage that the catalyst has to be separated from the reaction mixture. This separation step results in waste streams which have to be subjected to a complicated work-up. In addition, the homogeneous catalyst cannot be regenerated.

The disadvantages described do not occur in the heterogeneously catalyzed olefin oligomerization. Processes for oligomerizing olefins over nickel-comprising heterogeneous catalysts for the preparation of oligomers are described, for example, in DE 43 39 713 A1. That document discloses a process for oligomerizing unbranched C2-C6-olefins over a fixed-bed catalyst at superatmospheric pressure and elevated temperature, in which the catalyst comprises, as significant active constituents, from 10 to 70 % by weight of nickel oxide, from 5 to 30 % by weight of titanium dioxide and/or zirconium dioxide, from 0 to 20 % by weight of aluminum oxide and silicon oxide as balance. The oligomerization of butenes can be carried out with very good selectivity to linear products by this process.

The heterogeneously catalyzed process however has the disadvantage that the nickel containing catalyst deactivates over time, leading to lower conversion, lower selectivity with respect to dimers and higher branching, i.e. higher ISO indexes. From a cost perspective, it is desirable to regenerate deactivated catalysts and reuse them in further oligomerization reactions. One method for regeneration of used catalysts known in the art is calcination, i.e. heating the used catalyst in the presence of oxygen to temperatures of from 300°C to 750°C and burning off organic material on the surface of the catalyst. It is further known to impregnate the calcinated used catalyst with additional nickel as a further regeneration step. Document WO 2011/000697 A1 for example discloses a method for regenerating nickel containing catalysts that have been used for the oligomerization of olefins comprising the steps of calcination, impregnation with nickel and activation by a further temperature treatment. Positive results are reported for used catalysts that contained 5 to 50 % by weight of Ni, 5 to 30 % by weight Al₂O₃ and 30 to 80 % by weight SiO₂.

In practice, it turned out that methods of regeneration of used catalysts according to the prior art are differently suited for different kinds of catalysts. It was an object of the invention to provide a method for regenerating a used catalyst that increases the efficiency of the catalyst for olefin oligomerization reactions in terms of conversion, selectivity while maintaining a desired degree of branching.

This task is solved according to the invention by a process for regenerating a used catalyst according to claim 1 and a process for the production of oligomers according to claim 8. Advantageous variants of the processes are presented in claim 2 to claim 7 and claim 9.

### BRIEF DESCRIPTION

A first subject of the invention is a process for regenerating a used catalyst which has been used for the oligomerization of C3 to C6 olefins, the catalyst in the fresh state having had an active material composition of 10 to 70 % by weight of nickel(II) oxide (NiO), 5 to 30 % by weight titanium dioxide (TiO₂) and/or zirconium dioxide (ZrO₂), 0 to 20 % by weight aluminium(III) oxide (Al₂O₃) and the balance to 100 % by weight silicon dioxide (SiO₂), comprising the steps of a) calcining the used catalyst at a temperature of 300 to 600°C in the presence of an oxygen containing gas, b) applying nickel in an amount corresponding to from 1 to 5 % by weight of the used catalyst mass to the catalyst obtained in step a), and c) drying and calcining the catalyst obtained in step b).

A second subject of the invention is a process for the production of oligomers in which a feed mixture containing C3 to C6 olefins is fed to a reaction zone, wherein the oligomerization in the reaction zone takes place in the presence of a catalyst which has been regenerated by a process according to the invention. Preferably, the feed mixture contains C4 olefins.

It has been found that a regeneration of the used catalyst with small amounts of additional nickel showed an increase in conversion and a low branching degree in a subsequent process for producing oligomers of C3 to C6 olefins.

Other technical features may be readily apparent to one skilled in the art from the following description and claims.

### DEFINITIONS

"Active material" refers to the sum of the oxidic substances without the mass of graphite or other additives used to form the catalyst.

"ISO index" refers to a value that is calculated based on the composition of a mixture of isomers as the mean number of alkyl branches of the respective isomers. An isomer with no alkyl branches counts 0 (zero), an isomer with one alkyl branch counts 1 (one), an isomer with two alkyl branches counts 2 (two), an isomer with three alkyl branches counts 3 (three), etc. The calculation is based on the mass fractions of the respective isomers. For example, a mixture of isomers containing 35 % of isomers with no methyl branches, 25 % of isomers with one methyl branch and 40 % of isomers with two methyl branches results in an ISO index of 1.05.

"Oligomerization" refers to the chemical reaction of at least two olefins to form a new olefin where the number of carbon atoms of the new olefin is equal to the sum of the carbon atoms of the reaction partners. The new olefin is also termed "oligomer". Examples for oligomers are dimers, trimers and tetramers. The reaction partners may be of the same kind, for example C3 olefins only, C4 olefins only, C5 olefins only or C6 olefins only. The reaction partners may also be of different kind, for example a C3 olefin and a C5 olefin or a C4 olefin and a C6 olefin. The latter kind of reaction is also termed "co-oligomerization".

"Oxygen containing gas" refers to a gas that contains at least 5 vol.-% of oxygen. Preferably, the oxygen containing gas contains mainly oxygen and nitrogen, for example air. It is further preferred that the ratio of oxygen to nitrogen is chosen such that the heat tone occurring during treatment of the used catalyst is less than 50°C.

If not indicated otherwise, throughout this document "percent (%)" means weight percent (wt.-%) and the respective numbers are based on the total mass of the respective material.

"Used catalyst" refers to a nickel containing catalyst that has been used at least once for oligomerizing C3 to C6 olefins.

### PREFERRED EMBODIMENTS

Calcination of the used catalyst in process step a) may be performed in an oxygen-containing atmosphere, preferably under air. The preferred temperature range for calcination is from 300°C to 650°C, more preferably from 400°C to 600°C. Calcination may be performed in conventional furnaces, for example in a muffle furnace, a rotary kiln or a shaft furnace. The used catalyst has carbonaceous deposits. Consequently, carbon oxides and water are formed as oxidation products during calcination.

In process step b), nickel is applied to the catalyst obtained in step a). Preferably, nickel is applied as a nickel compound. As a nickel compound, all compounds of nickel are suitable, which can be converted into an oxidic form of nickel when heated in the presence of oxygen or oxygen-containing gas mixtures such as air under the calcination conditions. Preferably, water-soluble nickel salts, for example hydrated nickel nitrate or nickel salts having organic anions such as formate, oxalate, acetate, acetylacetonate or 2-ethylhexanoate are used as nickel compounds. Particularly preferred is hydrated nickel nitrate.

In a preferred embodiment of the regeneration process the application of nickel in step b) is carried out by impregnating the used catalyst with a nickel containing solution, in particular a nickel nitrate containing solution, for example a hydrated nickel nitrate solution. Preferably, the calcined catalyst is mixed with a solution containing the amount of nickel to be applied to the catalyst. In this case, the amount of the solution is advantageously selected so that it corresponds to 90 - 100 % of the water absorption capacity of the catalyst material.

The impregnated catalyst is dried and calcinated in process step c). Drying may be performed in air at a temperature of from 50 to 200°C. Calcination may be performed as described above in relation to step a). In a preferred embodiment of the regeneration process the drying in step c) takes place at a temperature of 80 to 160°C and the subsequent calcination takes place at a temperature of 300 to 600°C.

The regenerated catalyst may be further treated before using it in an oligomerization process again. In one embodiment, the catalyst is subjected to conditioning in a dry stream of an inert gas, preferably in a dry nitrogen stream, for example at atmospheric pressure and temperatures of from 50 to 500°C, preferably of from 100 to 250°C, to remove traces of moisture, which may originate from the air, for example, from the catalyst.

The used catalyst may have any form and geometrical structure suitable for the respective application. In a preferred embodiment of the invention the used catalyst is provided in the form of extrudates or tablets, more preferably in the form of tablets. In a preferred embodiment of the regeneration process the used catalyst has a mass fraction of carbon of 3 to 10 % by weight of the total mass of the catalyst.

The used catalyst has been used at least once for oligomerizing C3 to C6 olefins. Preferably, the used catalyst has already been regenerated at least once and used again for the oligomerization of C3 to C6 olefins.

Preferably, the catalyst in the fresh state had an active material composition of 40 to 60 % by weight NiO, 5 to 20 % by weight TiO₂ and/or ZrO₂, 0 to 10 % by weight Al₂O₃ and the balance to 100 % by weight SiO₂.

More preferably, the catalyst in the fresh state had an active material composition of 45 to 55 % by weight NiO, 8 to 18 % by weight TiO₂ and/or ZrO₂, 1 to 5 % by weight Al₂O₃ and the balance to 100 % by weight SiO₂.

### DETAILED DESCRIPTION

### Comparative Example 1

A sample of a used catalyst was taken from a reactor of an industrial butene dimerization plant. In the fresh state before the first use the catalyst had an active material composition of 46.5 % by weight NiO, 13.9 % by weight TiO₂, 3.3 % by weight Al₂O₃ and 36.3 % by weight SiO₂. The catalyst was provided in the form of 3x3 mm tablets that were produced using 97 % of active material and 3% of graphite as tableting aid. The catalyst had been regenerated once before and was in second use. The used catalyst contained 5.1 % carbon (C).

As a base case, the used catalyst was regenerated by calcination according to the prior art without the addition of further substances such as nickel. An amount of 600 grams (g) of used catalyst was heated in a rotary kiln at a rate of 5 Kelvin per minute (K/min) from room temperature to a temperature of 450°C. This temperature was maintained for two hours with an air supply to the rotary kiln of 100 liters per hour (I/h). After this time span the catalyst was allowed to passively cool down without additional measures. This procedure was repeated two times and the resulting catalyst samples were mixed to obtain about 1.7 kilograms (kg) of calcinated catalyst. The water uptake of the catalyst was 0.73 milliliters per gram (ml/g). Its carbon content was reduced from 5.1 % to 3.6 %.

### Examples according to the invention

In a series of experiments parts of the calcinated catalyst samples of the base case were impregnated with different amounts of nickel.

In a first experiment according to the invention 55.7 g of an aqueous nickel nitrate solution with a nickel oxide content in the aqueous solution of 17.6 % were diluted with 167 g of water. This resulted in an amount of 208 milliliters (ml) of impregnating solution. An amount of 300 g of the calcinated catalyst of the base case were intensely mixed with these 208 ml of the impregnating solution in a porcelain dish to a water uptake of 95 %. This corresponds to an amount of additional nickel of 2.5 % of the mass of the used catalyst.

The resulting impregnated tablets were dried for 16 hours (h) in a convection oven at 120°C. After the drying process the tablets were calcinated by heating in a muffle furnace at a rate of 5 Kelvin per minute (K/min) from room temperature to a temperature of 450°C. This temperature was maintained for two hours. The total amount of nickel of the regenerated catalyst was determined by atomic spectroscopy measurements to 37 %. For the measurements, two aliquots of about 0.10 to 0.15 g of the sample were digested with concentrated inorganic acids under heating. The mineralized residues were dissolved in diluted hydrochloric acid to 100 mL. The thus prepared solutions were analyzed via inductively coupled plasma optical emission spectroscopy (ICP-OES) for their Nickel content using an external calibration. The reported result was the average value from both duplicates. A blank sample was prepared in analogous manner.

In a second experiment according to the invention 22.3 g of the aqueous nickel nitrate solution with a nickel oxide content in the aqueous solution of 17.6 % were diluted with water, resulting in an amount of 138 ml of impregnating solution. An amount of 200 g of the calcinated catalyst of the base case were intensely mixed with the impregnating solution. All other parameters were the same as for the first experiment. Regenerated catalysts were obtained with an additional amount of nickel of 1.5 %.

In a third experiment according to the invention 60.4 g of the aqueous nickel nitrate solution with a nickel oxide content in the aqueous solution of 17.6 % were diluted with water, resulting in an amount of 138 ml of impregnating solution. An amount of 200 g of the calcinated catalyst of the base case were intensely mixed with the impregnating solution. All other parameters were the same as for the first experiment. Regenerated catalysts were obtained with an additional amount of nickel of 4 %.

### Comparative Example 2

In a further experiment the concentration of nickel in the impregnating solution was further increased to obtain a regenerated catalyst with an additional amount of nickel of 6 %. 92.5 g of the aqueous nickel nitrate solution were diluted with water, resulting in an amount of 138 ml of impregnating solution. An amount of 200 g of the calcinated catalyst of the base case were intensely mixed with the impregnating solution. All other parameters were the same as for the first experiment.

Samples of the different regenerated catalysts were tested in a reactor system on technical scale for the dimerization of butenes. Two reactors were connected in series. The first reactor was fed by a feed stream of raffinate-II. As the feed was taken from a real plant, the feed compositions varied among the experiments. Table 1 shows the composition in percent by weight of the three feed streams used in the experiments. Further hydrocarbons like 1,3-butadiene, propene, propane, cyclopropane, propadiene, methylcyclopropane, vinylacetylene, pentenes and pentanes were present in trace amounts. Water and oxygenates were removed by flowing the feed streams over a molar sieve (3A) and a purification catalyst before entering the reactor system.

**Table 1:**

| **Component** | **Feed 1** | **Feed 2** | **Feed 3** |
|---|---|---|---|
| isobutene | 2.4 | 2.7 | 2.9 |
| 1-butene | 45.8 | 47.5 | 42.5 |
| cis-2-butene | 11.4 | 12.2 | 10.7 |
| trans-2-butene | 20.9 | 20.6 | 18.0 |
| isobutane | 4.8 | 3.9 | 5.6 |
| n-butane | 14.6 | 13.1 | 20.0 |

The outlet of the first reactor was fed to the inlet of the second reactor. A part of the outlet of the second reactor was recycled to the second reactor. The remaining part of the outlet of the second reactor was withdrawn as product stream.

Each reactor comprised a reaction tube with an inner diameter of 15 mm and a tube length of 2200 mm. The two reaction tubes were each filled with 125 ml of catalyst, bounded at each tube end by a layer of steatite balls. The internal tube temperatures were measured via multiple thermocouples. The operation of the reactors was quasi-isothermal.

Each reactor was equipped with 125 ml of regenerated catalyst. For this purpose, the catalysts obtained in the examples described above were provided as tablets with a size of 3x3 mm in each case. Before each run the reactor system was flown through by an inert nitrogen stream for 12 hours at a temperature of 250°C. For each reaction experiment a stream of 250 g/h of raffinate-II feed was fed to the first reactor. The recycle stream over the second reactor was set to 1.5 kg/h. The temperatures in both reactors were set to 80°C at a pressure inside the reactor of 30 bar (abs).

The compositions of the feed stream, the outlet stream of the first reactor and the product stream of the second reactor were determined with an on-line gas chromatograph with two columns for the simultaneous determination of C4 isomers and of olefins with 4, 8, 12 and more carbon atoms. The ISO index was determined by additional off-line analytic in a hydrogenating gas chromatograph.

Table 2 shows the results of the experiments with five different regenerated catalysts. The percentage of added nickel is given in the first column of Table 2. The numbers in column two refer to the feed compositions given in Table 1. The values in columns three and five show the conversion of C4 components in the respective reactor. The values in columns four and six show the selectivity to C8 components in the respective reactor. The rightmost column shows the value of the ISO index of the C8 product obtained from the second reactor for each experiment. The first row of each pair of results refers to analyses taken after a reactor runtime of approximately two days, and the second row of each pair of results refers to analyses taken after a reactor runtime of approximately nine days.

**Table 2:**

| **First reactor** | | | | **Second reactor** | | |
|---|---|---|---|---|---|---|
| **Add. Ni (%)** | **Fee d** | **C4 Conv (%)** | **C8 Sel (%)** | **C4 Conv (%)** | **C8 Sel (%)** | **C8 ISO index** |
| 0 | 3 | 45.6 | 66.4 | 53.2 | 64.8 | 1.13 |
| 0 | 3 | 44.2 | 67.7 | 52.0 | 66.0 | 1.04 |
| 1.5 | 2 | 49.9 | 72.4 | 57.0 | 69.3 | 1.00 |
| 1.5 | 2 | 47.6 | 74.9 | 55.3 | 72.0 | 0.99 |
| 2.5 | 3 | 50.7 | 68.1 | 59.5 | 64.9 | 0.95 |
| 2.5 | 3 | 50.0 | 70.1 | 58.6 | 67.0 | 1.03 |
| 4 | 1 | 42.5 | 74.2 | 51.3 | 71.7 | 1.01 |
| 4 | 1 | 41.4 | 75.2 | 50.2 | 73.0 | 1.00 |
| 6 | 2 | 35.4 | 75.0 | 35.3 | 75.2 | 1.06 |
| 6 | 2 | 36.1 | 75.5 | 36.2 | 75.6 | 1.09 |

As expected, a regeneration of the used catalyst by calcination only yielded moderate values for the butene (C4) conversion, isooctene selectivity and a rather high C8 ISO index. Reaction experiments with the three samples of regenerated catalysts with small amounts (1.5 %, 2.5 %, 4 %) of additional nickel showed an increase in isooctene selectivity and a branching degree of the isooctene (C8 ISO index) in the desired range of 0.95 to 1.05. The butene conversion for the catalysts with small amounts of additional nickel was similar or higher compared to the catalyst without nickel addition. The addition of a small amount of nickel to the used catalyst that is already rich in nickel turned out to be beneficial. The addition of even more nickel (6 %) however, showed a much lower conversion of butenes and an undesired increase in the formation of branched isooctenes, which can be deducted from the rather high ISO index.

## Claims

1. A process for regenerating a used catalyst which has been used for the oligomerization of C3 to C6 olefins, the catalyst in the fresh state having had an active material composition of 10 to 70 % by weight of NiO, 5 to 30 % by weight TiO₂ and/or ZrO₂, 0 to 20 % by weight Al₂O₃ and the balance to 100 % by weight SiO₂, comprising the steps of:
a) calcining the used catalyst at a temperature of 300 to 600°C in the presence of an oxygen containing gas,
b) applying nickel in an amount corresponding to from 1 to 5 % by weight of the used catalyst mass to the catalyst obtained in step a), and
c) drying and calcining the catalyst obtained in step b).

2. The process of claim 1, **characterized in that** the application of nickel in step b) is carried out by impregnating the used catalyst with a nickel containing solution, in particular a nickel nitrate containing solution.

3. The process of claim 1 or 2, **characterized in that** the drying in step c) takes place at a temperature of 80 to 160°C and the subsequent calcination takes place at a temperature of 300 to 600°C.

4. The process of any one of claims 1 to 3, **characterized in that** the used catalyst has a mass fraction of carbon of 3 to 10 % by weight.

5. The process of any one of claims 1 to 4, **characterized in that** the used catalyst has already been regenerated at least once and used again for the oligomerization of C3 to C6 olefins.

6. The process of any one of claims 1 to 5, **characterized in that** the catalyst in the fresh state had an active material composition of 40 to 60 % by weight NiO, 5 to 20 % by weight TiO2 and/or ZrO2, 0 to 10 % by weight Al2O3 and the balance to 100 % by weight SiO2.

7. The process of any one of claims 1 to 5, **characterized in that** the catalyst in the fresh state had an active material composition of 45 to 55 % by weight NiO, 8 to 18 % by weight TiO2 and/or ZrO2, 1 to 5 % by weight Al2O3 and the balance to 100 % by weight SiO2.

8. A process for the production of oligomers in which a feed mixture containing C3 to C6 olefins is fed to a reaction zone, **characterized in that** the oligomerization in the reaction zone takes place in the presence of a catalyst which has been regenerated by a process according to any one of claims 1 to 7.

9. The process for the production of oligomers of claim 8, **characterized in that** the feed mixture contains C4 olefins.
